# EUROPEAN PATENT APPLICATION

(11) **EP 4 789 720 A2**
(43) Date of publication of application: **12.08.2026**
(21) Application number: 26187434.1
(22) Date of filing: 15.05.2023
(51) Int. Cl.: A61P 25/28

(54) **NASAL FORMULATIONS OF FORALUMAB**

(30) Priority: 13.05.2022 US 202263341856 P
(62) Document number(s) of the earlier application(s) in accordance with Art. 76 EPC: 23727296.8 / 4 522 115
(71) Applicant: Tiziana Life Sciences PLC, London Greater London SW1Y 4LB (GB)
(72) Inventor: Jacob, Jules S., Perkasie, 18942 (US); PALEJWALA, Vaseem A., Scotch Plains, 07076 (US); SHAILUBHAI, Kunwar, Line Lexington, 18932 (US)
(74) Representative: Abel & Imray LLP

(57) **Abstract**

The present disclosure provides a unit dose device for the intranasal administration of a liquid formulation comprising foralumab, wherein 10 µg to 100 µg of foralumab is delivered per dose in a volume of 100 µl, and wherein the delivered droplet size is between 10 µm and 100 µm or between 25 µm and 250 µm. Also provided is a liquid formulation comprising an anti-cd3 antibody for use in treating or alleviating a symptom of an autoimmune disease, an inflammatory disorder, a neurodegenerative disease or cancer, wherein the formulation is for nasal administration. The anti-CD3 antibody comprises CDRs having the amino acid sequences of SEQ ID NOs: 1, 3, 4, 5, 6 and 7.

## Description

### FIELD OF THE INVENTION

This invention relates to nasal formulations, dosages, and dosing regimens of the anti-CD3 antibody foralumab.

### BACKGROUND

Antibodies to the CD3 epsilon signaling molecule of the T-cell receptor complex have proven to be useful as immunosuppressants and in the treatment of autoimmune disorders. Thus, improved methods of preparing anti-CD3 antibodies, methods of purifying anti-CD3 antibodies and pharmaceutical formulations containing anti-CD3 antibodies would be useful.

### SUMMARY OF THE INVENTION

The present disclosure provides formulation, dosages, and dosing regimens for monoclonal antibodies specifically directed against CD3. The formulations of the present disclosure include the anti-CD3 antibody, Foralumab. The anti-CD3 antibody formulation is a nasal formulation.

In some embodiments, the anti-CD3 antibody of the formulations of the disclosure comprises a heavy chain complementarity determining region 1 (CDRH1) comprising the amino acid sequence GYGMH (SEQ ID NO: 1), a heavy chain complementarity determining region 2 (CDRH2) comprising the amino acid sequence VIWYDGSKKYYVDSVKG (SEQ ID NO: 3), a heavy chain complementarity determining region 3 (CDRH3) comprising the amino acid sequence QMGYWHFDL (SEQ ID NO: 4), a light chain complementarity determining region 1 (CDRL1) comprising the amino acid sequence RASQSVSSYLA (SEQ ID NO: 5), a light chain complementarity determining region 2 (CDRL2) comprising the amino acid sequence DASNRAT (SEQ ID NO: 6), and a light chain complementarity determining region 3 (CDRL3) comprising the amino acid sequence QQRSNWPPLT (SEQ ID NO: 7).

In some embodiments, the anti-CD3 antibody of the formulations of the disclosure comprises a variable heavy chain (VH) amino acid sequence comprising the amino acid sequence of SEQ ID NO: 8 and a variable light (VL) chain amino acid sequence comprising the amino acid sequence of SEQ ID NO: 9. In other aspects, the anti-CD3 antibody comprises a heavy chain amino acid sequence comprising the amino acid sequence of SEQ ID NO: 10 and a light chain amino acid sequence comprising the amino acid sequence of SEQ ID NO: 11.

Provided by the disclosure is a formulation for nasal delivery comprising 0.25 or 0.5 mg/mL foralumab, 3.4 mg/mL sodium acetate, 0.20 mg/ml polysorbate 80 and 7.31 mg/ ml sodium chloride.

In other aspects, the disclosure provides a unit dose device for the intranasal administration of foralumab. In some embodiments, the unit dose device contains a nasal atomization device and 0.25 or 0.5 mg/mL foralumab, 3.4 mg/mL sodium acetate, 0.20 mg/ml polysorbate 80, and 7.31 mg/ ml sodium chloride.

In some embodiments, the unit dose device is capable of delivering 100 µL of the formulation. In some embodiments, the nasal atomization device is an Aptar UniDose device. In some embodiments, the nasal administration device delivers a droplet size of the formulation of about between 10 µm and 100 µm.

In some embodiments, the nasal atomization devise is a Gerresheimer device. In some embodiments, the nasal administration device delivers a droplet size of the formulation of about between 25 µm and 250 µm.

In some embodiments, the foralumab concentration in the formulation is within 3%, within 5%, or within 10 % of the starting concentration after store at about 2 °C to about 8 °C for at least about one month, at least about three months, at least about 6 months, at least about 12 months, at least about 18 months, at least about 2 years, at least about 3 years or at least about 5 years.

In some embodiments, the formulation contains less than 1%, less than 1.5%, less than 2% or less than 2.5% aggregation after store at about 2 °C to about 8 °C for at least about one month, at least about three months, at least about 6 months, at least about 12 months, at least about 18 months, at least about 2 years, at least about 3 years or at least about 5 years.

In some embodiments, the pH of the formulation remains within 0.1, 0.2 or 0.3 of starting value after store at about 2 °C to about 8 °C for at least about one month, at least about three months, at least about 6 months, at least about 12 months, at least about 18 months, at least about 2 years, at least about 3 years or at least about 5 years.

In some embodiments, the potency of the foralumab in the nasal anti-CD3 antibody formulation remains within 10%, within 15%, within 20%, within 25%, within 30%, or within 35% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

The invention further provides methods of treating or alleviating a symptom of autoimmune disease, an inflammatory disorder, a neurodegenerative disease or cancer by administering to a subject in need thereof a formulation according to the invention. For example, the present disclosure provides methods of treating or alleviating a symptom of multiple sclerosis or Alzheimer's Disease.

Other features and advantages of the invention will be apparent from and encompassed by the following detailed description and claims.

### DETAILED DESCRIPTION

The present disclosure provides nasal formulations and dosing for monoclonal antibodies, *e*.*g*., fully human monoclonal antibodies that specifically bind the CD3 epsilon chain (CD3ε). Specifically, the disclosure provides nasal formulations of anti-CD3ε antibodies useful to target tissue specific immunomodulation. Unlike systemic (*e.g*., intravenous) administration of anti-CD3 antibodies, the formulation of the present invention is believed to minimize off-target immunosuppression. The formulations provided herein are useful in treating or alleviating a symptom of autoimmune diseases, inflammatory disorders, neurodegenerative disorders and cancer.

### CD3 Antibodies

The present disclosure provides formulation for nasal delivery of Foralumab, an antibody which specifically binds to the CD3 epsilon chain (CD3ε).

Foralumab comprises a heavy chain complementarity determining region 1 (CDRH1) comprising the amino acid sequence GYGMH (SEQ ID NO: 1), a heavy chain complementarity determining region 2 (CDRH2) comprising the amino acid sequence VIWYDGSKKYYVDSVKG (SEQ ID NO: 3), a heavy chain complementarity determining region 3 (CDRH3) comprising the amino acid sequence QMGYWHFDL (SEQ ID NO: 4), a light chain complementarity determining region 1 (CDRL1) comprising the amino acid sequence RASQSVSSYLA (SEQ ID NO: 5), a light chain complementarity determining region 2 (CDRL2) comprising the amino acid sequence DASNRAT (SEQ ID NO: 6), and a light chain complementarity determining region 3 (CDRL3) comprising the amino acid sequence QQRSNWPPLT (SEQ ID NO: 7).

Foralumab comprises a variable heavy chain amino acid sequence comprising QVQLVESGGGVVQPGRSLRLSCAASGFKFSGYGMHWVRQAPGKGLEWVAVIWYDGS KKYYVDSVKGRFTISRDNSKNTLYLQMNSLRAEDTAVYYCARQMGYWHFDLWGRG TLVTVSS (SEQ ID NO: 8) and a variable light chain amino acid sequence comprising EIVLTQSPATLSLSPGERATLSCRASQSVSSYLAWYQQKPGQAPRLLIYDASNRATGIPA RFSGSGSGTDFTLTISSLEPEDFAVYYCQQRSNWPPLTFGGGTKVEIK (SEQ ID NO: 9).

Foralumab comprises a heavy chain amino acid sequence comprising: 
QVQLVESGGGVVQPGRSLRLSCAASGFKFSGYGMHWVRQAPGKGLEWVAVIWYDGS
KKYYVDSVKGRFTISRDNSKNTLYLQMNSLRAEDTAVYYCARQMGYWHFDLWGRG
TLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKSCDKTHTCPP
CPAPEAEGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 10)
and a light chain amino acid sequence
comprising:EIVLTQSPATLSLSPGERATLSCRASQSVSSYLAWYQQKPGQAPRLLIYDAS NRATGIPARFSGSGSGTDFTLTISSLEPEDFAVYYCQQRSNWPPLTFGGGTKVEIKRTVA APSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC (SEQ ID NO: 11).

Foralumab is also referred to herein as NI-0401, or 28F11-AE. (*See e.g.,* Dean Y, Dépis F, Kosco-Vilbois M. "Combination therapies in the context of anti-CD3 antibodies for the treatment of autoimmune diseases." Swiss Med Wkly. (2012), the contents of which are hereby incorporated by reference in its entirety).

In some embodiments, the anti-CD3 antibody is a fully human antibody or a humanized antibody. In some embodiments, the anti-CD3 antibody formulation includes a full length anti-CD3 antibody. In alternative embodiments, the anti-CD3 antibody formulation includes an antibody fragment that specifically binds CD3. In some embodiments, the anti-CD3 antibody formulation includes a combination of full-length anti-CD3 antibodies and antigen binding fragments that specifically bind CD3.

In some embodiments, the antibody or antigen-binding fragment thereof that binds CD3 is a monoclonal antibody, a domain antibody, a single chain, a Fab fragment, a F(ab')₂ fragment, a scFv, a scAb, a dAb, a single domain heavy chain antibody, or a single domain light chain antibody. In some embodiments, the antibody or antigen-binding fragment thereof that binds CD3 is a mouse, other rodent, chimeric, humanized or fully human monoclonal antibody.

Optionally, the anti-CD3 antibody or antigen binding fragment thereof used in the formulations of the disclosure includes at least one an amino acid mutation. Typically, the mutation is in the constant region. In some embodiments, the mutation results in an antibody that has an altered effector function. An effector function of an antibody is altered by altering, *i.e.,* enhancing or reducing, the affinity of the antibody for an effector molecule such as an Fc receptor or a complement component. For example, the mutation may result in an antibody that is capable of reducing cytokine release from a T-cell. In some embodiments, the mutation is in the heavy chain at amino acid residue 234, 235, 265, or 297 or combinations thereof (with numbering according to the Kabat numbering scheme for immunoglobulins)Preferably, the mutation results in an alanine residue at position 234, 235, 265 and/or 297, or a glutamate residue at position 235, or a combination thereof. Examples of mutations that may be present in the anti-CD3 antibody used in the formulations described herein include L²³⁴ L²³⁵ → A²³⁴ E²³⁵, L²³⁴ L²³⁵ → A²³⁴ A²³⁵, L²³⁵ → E²³⁵, N²⁹⁷ → A²⁹⁷, and D²⁶⁵ → A²⁶⁵.

Preferably, the anti-CD3 antibody used in the formulations provided herein contains one or more mutations that prevent heavy chain constant region-mediated release of one or more cytokine(s) *in vivo.*

In some embodiments, the anti-CD3 antibody or antigen binding fragment thereof used in the formulations of the disclosure is a fully human antibody. The fully human anti-CD3 antibodies used in the formulations provided herein may comprise, for example, a L²³⁴ L²³⁵ → A²³⁴ E²³⁵ mutation in the Fc region, such that cytokine release upon exposure to the anti-CD3 antibody is significantly reduced or eliminated. The L²³⁴ L²³⁵ → A²³⁴ E²³⁵ mutation in the Fc region of the anti-CD3 antibodies used in the formulations provided herein is believed to reduce or eliminate cytokine release when the anti-CD3 antibodies are exposed to human leukocytes, whereas antibodies comprising other mutations (such as L²³⁴ L²³⁵ → A²³⁴ A²³⁵, L²³⁵ → E²³⁵, N²⁹⁷ → A²⁹⁷, and D²⁶⁵ → A²⁶⁵) maintain significant cytokine release capacity. For example, a significant reduction in cytokine release may be determined by comparing the release of cytokines upon exposure to the anti-CD3 antibody having a L²³⁴ L²³⁵ → A²³⁴ E²³⁵ mutation in the Fc region to level of cytokine release upon exposure to another anti-CD3 antibody having no mutation or having one or more other mutations (such as L²³⁴ L²³⁵ → A²³⁴ A²³⁵, L²³⁵ → E²³⁵, N²⁹⁷ → A²⁹⁷, and D²⁶⁵ → A²⁶⁵).

The term "cytokine" refers to all human cytokines known within the art that bind extracellular receptors expressed on the cell surface and thereby modulate cell function, including but not limited to IL-2, IFN-gamma, TNF-a, IL-4, IL-5, IL-6, IL-9, IL-10, and IL-13.

The anti-CD3 antibody used in the formulations described herein may be of any class of immunoglobulin, i.e., the anti-CD3 antibody may be an IgG, IgM, IgA, IgE or IgD antibody. The anti-CD3 antibody may also be of any subclass of immunoglobulins, such as IgG1, IgG2, IgG3, or IgG4. In some embodiments, the anti-CD3 antibody is an IgG1 antibody.

### Formulations

The anti-CD3 antibody formulation of this disclosure can be a liquid. For example, the liquid formulation may be aqueous.

The anti-CD3 antibody formulation of this disclosure may include one or more salts (a buffering salt), one or more polyols and/or one or more excipients. The formulations of the present disclosure may also contain buffering agents and/or preservatives. The anti-CD3 antibody formulation may be buffered in a solution at a pH in the range of about 4 to 8; in the range of about 4 to 7; in the range of about 4 to 6; in the range of about 5 to 6; or in the range of about 5.5 to 6.5. In preferred embodiments, the antibody formulation is buffered in a solution at a pH of about 5.5.

Examples of salts that may be present in the formulations provided herein include those prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid, and the like. Such salts can also be prepared as alkaline metal or alkaline earth salts, such as sodium, potassium or calcium salts. Examples of buffering agents that may be present in the formulations of the disclosure include phosphate, citrate, acetate, and 2-(N-morpholino) ethanesulfonic acid (MES).

The formulations of the present invention may include a buffer system. As used in this application, the terms "buffer" or "buffer system" describes a compound that, usually in combination with at least one other compound, is capable of neutralizing, within limits, either acids or bases (alkali) with relatively little or no change in the original pH.

Buffers that may be present in the formulations of the present disclosure include borate buffers, phosphate buffers, calcium buffers, and combinations and mixtures thereof. Borate buffers that may be present in the formulations of the disclosure include, for example, boric acid and its salts, for example, sodium borate or potassium borate. Borate buffers also include compounds such as potassium tetraborate or potassium metaborate that produce borate acid or its salt in solutions.

A phosphate buffer system includes one or more monobasic phosphates, dibasic phosphates and the like. Particularly useful phosphate buffers are those selected from phosphate salts of alkali and/or alkaline earth metals. Examples of suitable phosphate buffers that may be present in the formulations of the disclosure include one or more of sodium dibasic phosphate (Na2HPO4), sodium monobasic phosphate (NaH2PO4) and potassium monobasic phosphate (KH2PO4). The phosphate buffer components may be used in amounts from 0.01% or to 0.5% (w/v), calculated as phosphate ion.

Other known buffer compounds can optionally be added to the formulations of the disclosure, including for example, citrates, sodium bicarbonate, TRIS, and the like. Other ingredients in the formulation, while having other functions, may also affect the buffer capacity. For example, EDTA, often used as a complexing agent, can have a noticeable effect on the buffer capacity of a solution.

Preferred salts that may be used in the formulation of the disclosure include sodium chloride, sodium acetate, sodium acetate trihydrate and sodium citrate.

In some embodiments, the concentration of salt in the formulations according to the disclosure is between about 10 mM and about 500 mM, between about 25 m and 250 mM, between about 25 nM and 150 mM.

In some embodiments, the sodium acetate trihydrate is present in a formulation provided herein at a concentration in the range of about 10 mM to about 100 mM. For example, the sodium acetate trihydrate may be present in a formulation provided herein at a concentration of about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 9 mM 5 or about 100 mM. In some embodiments the sodium acetate trihydrate is present in a formulation provided herein at a concentration of 25 mM.

In some embodiments, sodium chloride is present in a formulation provided herein at a concentration in the range of about 50 mM to about 500 mM. In some embodiments, the sodium chloride is present in a formulation provided herein at a concentration of about 50 mM, about 55 mM, about 60 mM, about 65 mM, about 70 mM, about 75 mM, about 80 mM, about 85 mM, about 90 mM, about 95 mM, about 100 mM, about 125 mM, about 150 mM, about 175 mM, about 200 mM, about 225 mM, about 250 mM, about 275 mM, about 300 mM, about 325 mM, about 350 mM, about 375 mM, about 400 mM, about 425 mM, about 450 mM, about 475 mM or about 500 mM. In some embodiments, the sodium chloride is present in a formulation provided herein at a concentration of about 125mM.

In some embodiments, the sodium citrate is present in a formulation provided herein at a concentration in the range of about 10 mM to about 100 mM. In some embodiments, the sodium citrate is present in a formulation provided herein at a concentration of about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM or about 100 mM. In some embodiments, the sodium citrate is present in a formulation provided herein at a concentration in the range of about 25 mM to about 50 mM.

In some embodiments, a formulation provided herein comprises sodium acetate trihydrate at a concentration in the range of about 25 mM to about 100 mM and sodium chloride at a concentration in the range of about 150 mM to about 500 mM. In some embodiments, the formulation comprises about 25 mM sodium acetate trihydrate and about 150 mM sodium chloride.

In some embodiments, the formulation comprises one or more polyols as a bulking agent and/or stabilizing excipients. Polyols include for example, trehalose, mannitol, maltose, lactose, sucrose, sorbitol, or glycerol. The polyols is at a concentration in the range of about 0.1% to about 50% or about 5% to about 25%. For example, the polyol may be present in a formulation provided herein at a concentration of about 1%, about 2%, about 3%, about 4%, about 5%, about 10%, about 15%, about 20%, about 25%, about 30%, about 35%, about 40%, about 45% or about 50%

In some embodiments, the formulation comprises one or more excipients and/or surfactants to suppress or otherwise reduce antibody aggregation. Suitable excipients to reduce antibody aggregation include, for example, a surfactant such as, Polysorbate 20 or Polysorbate 80. In some embodiments, the Polysorbate 20 or Polysorbate 80 is present at a concentration in the range of about 0.01% to 1% or about 0.01% to about 0.05%. In some embodiments, the Polysorbate 20 or Polysorbate 80 is present in a formulation provided herein at a concentration of about 0.01 %, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%. about 0.9%, or about 1.0%.

In some embodiments, the surfactant is Polysorbate 80 and is present in a formulation described herein at a concentration in the range of about 0.01% to about 0.05%. In some embodiments, the Polysorbate 80 is present in a formulation provided herein at a concentration of about 0.02%.

In some embodiments, a formulation provided herein comprises one or more excipients to increase the stability of the antibody. In some embodiments, the excipient to increase stability is human serum albumin. In some embodiments, the human serum albumin is present in a formulation provided herein at a concentration in the range of about 1 mg to about 5 mg.

Suitable amino acids that may be present in a formulation described herein include for example, leucine, arginine, histidine, or combinations thereof.

Examples of antimicrobial agents that may be present in a formulation described herein include, for example, benzalkonium chloride, benzethonium chloride, benzoic acid, benzyl alcohol, butyl paraben, cetylpyridinium chloride, cresol, chlorobutanol, dehydroacetic acid, ethylparaben, methylparaben, phenol, phenylethyl alcohol, phenoxyethanol, phenylmercuric acetate, phenylmercuric nitrate, potassium sorbate, propylparaben, sodium benzoate, sodium dehydroacetate, sodium propionate, sorbic acid, thimersol, thymo, or mixtures thereof.

Examples of antioxidants that may be present in a formulation described herein include, for example, ascorbic acid, BHA, BHT, EDTA, or mixture thereof.

In some embodiments, the anti-CD3 antibody formulation described herein is a nasal formulation. In some embodiments, the nasal anti-CD3 antibody formulation is an aerosol formulation. In some embodiments, the nasal anti-CD3 antibody formulation is suitable for once daily administration. In some embodiments, the nasal anti-CD3 antibody formulation provides for aerosol of an anti-CD3 antibody at a dosage in the range of about 10 µg to 100 µg per single administration. In some embodiments, the nasal anti-CD3 antibody formulation provides for delivery of aerosol of an anti-CD3 antibody fragment at a dose of about 25 µg to about 50 µg per administration. In some embodiments, the administration is administered to one nostril or alternatively split between both nostrils.

In some embodiments, the formulation for nasal delivery comprises 0.25 mg/ml foralumab, 3.4 mg/mL sodium acetate, 0.20 mg/ml polysorbate 80 and 7.31 mg/ml sodium chloride. In some embodiments, the formulation for nasal delivery consists essentially of 0.25 mg/ml foralumab, 3.4 mg/mL sodium acetate, 0.20 mg/ml polysorbate 80 and 7.31 mg/ml sodium chloride.

In some embodiments, the formulation for nasal delivery comprises 0.5 mg/ml foralumab, 3.4 mg/mL sodium acetate, 0.20 mg/ml polysorbate 80 and 7.31 mg/ml sodium chloride. In some embodiments, the formulation for nasal delivery consists essentially of 0.5 mg/ml foralumab, 3.4 mg/mL sodium acetate, 0.20 mg/ml polysorbate 80 and 7.31 mg/ml sodium chloride.

In some embodiments, the osmolality of the formulation is about 800-950 (e.g., about 825-925) mOsm/kg.

In some embodiments, the average droplet size of the delivered formulation is between 10 µm and 250 µm. For example, the droplet size may be between 10 µm and 100 µm or between 25 µm and 250 µm.

In some embodiments, the nasal anti-CD3 antibody formulation is suitable for storage at about 2 °C to about 4 °C. In some embodiments, the nasal anti-CD3 antibody formulation is suitable for storage at about 2 °C to about 8 °C. In some embodiments, the nasal anti-CD3 antibody formulation is suitable for storage at about 5 °C. In some embodiments, the nasal anti-CD3 antibody formulation is stored in a sealed vial or other suitable container. In some embodiments, the nasal anti-CD3 antibody formulation is stored in a sealed vial or other suitable container at about 2 °C to about 4 °C.

In some embodiments, the antibody concentration in the nasal anti-CD3 antibody formulation is within 3% of the starting concentration after storage at about 2 °C to about 8 °C (e.g., about 5 °C) for at least about one month, at least about three months, at least about 6 months, at least about 12 months, at least about 18 months, at least about 2 years, at least about 3 years or at least about 5 years. "Starting or Label concentration" in this context refers to the concentration after manufacture but before storage.

In some embodiments, the antibody concentration in the nasal anti-CD3 antibody formulation is within 5% of the starting concentration after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the antibody concentration in the nasal anti-CD3 antibody formulation is within 10% of the starting concentration after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the nasal anti-CD3 antibody formulation contains less than 1.5% aggregation after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the nasal anti-CD3 antibody formulation contains less than 2% aggregation after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the nasal anti-CD3 antibody formulation contains less than 2.5% aggregation after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the nasal anti-CD3 antibody formulation contains less than 3% aggregation after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the pH of the nasal anti-CD3 antibody formulation remains within 0.1 of the starting pH after storage at about 2 °C to about 8 °C (e.g., about 5 °C) for at least about one month, at least about three months, at least about 6 months, at least about 12 months, at least about 18 months, at least about 2 years, at least about 3 years or at least about 5 years. "starting pH" in this context means the pH after manufacture but before storage.

In some embodiments, the pH of the nasal anti-CD3 antibody formulation remains within 0.2 of the starting pH after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the pH of the nasal anti-CD3 antibody formulation remains within 0.3 of the starting pH after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the pH of the nasal anti-CD3 antibody formulation remains within 0.4 of the starting pH after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the pH of the nasal anti-CD3 antibody formulation remains within 0.5 of the starting pH after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the appearance (including, e.g. cloudiness and colour) of the nasal anti-CD3 antibody formulation remains comparable to the appearance immediately after manufacture after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the impurities present in the nasal anti-CD3 antibody remain within 1% of the starting impurity level after storage at about 2 °C to about 8 °C (e.g., about 5 °C) for at least about one month, at least about three months, at least about 6 months, at least about 12 months, at least about 18 months, at least about 2 years, at least about 3 years or at least about 5 years. "Starting impurity level" here means the concentration of impurities measured after manufacture but before storage.

In some embodiments, the total aerobic microbial count in the nasal anti-CD3 antibody formulation remains below 100 cfu/g after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the total yeast and mold count in the nasal anti-CD3 antibody formulation remains below 10 cfu/g after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the total S. aureus count in the nasal anti-CD3 antibody formulation remains below 1g after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the total Ps. Aerugosina count in the nasal anti-CD3 antibody formulation remains below 1g after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the mean pI value of the main cIEF peak of the nasal anti-CD3 antibody formulation remains within 0.05 of the starting pI value after storage at about 2 °C to about 8 °C (e.g., about 5 °C) for at least about one month, at least about three months, at least about 6 months, at least about 12 months, at least about 18 months, at least about 2 years, at least about 3 years or at least about 5 years. "Starting pI value" here means the pI value of the main peak after manufacture but before storage.

In some embodiments, the mean pI value of the main cIEF peak of the nasal anti-CD3 antibody formulation remains within 0.1 of the starting pI value after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the mean pI value of the main cIEF peak of the nasal anti-CD3 antibody formulation remains within 0.2 of the starting pI value after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the potency of the antibody in the nasal anti-CD3 antibody formulation remains within 10% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years. "Starting potency" here means the antibody potency measured after manufacture but before storage. Antibody potency may be determined by lymphocyte proliferation assays or cell activation assays.

In some embodiments, the potency of the antibody in the nasal anti-CD3 antibody formulation remains within 15% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the potency of the antibody in the nasal anti-CD3 antibody formulation remains within 20% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years. In some embodiments, the potency of the antibody in the nasal anti-CD3 antibody formulation remains within 25% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the potency of the antibody in the nasal anti-CD3 antibody formulation remains within 30% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

In some embodiments, the potency of the antibody in the nasal anti-CD3 antibody formulation remains within 35% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.

The nasal formulation A formulation for nasal delivery having 0.25 or 0.5 mg/mL foralumab, 3.4 mg/mL sodium acetate, 0.20 mg/mL polysorbate 80 and 7.31 mg/mL sodium chloride upon storage for three months, 6 months or 12 months at about 2 °C to about 8 °C has the following characteristics: being substantially free of microbial contamination; being substantially free of protein aggregates; retains at least 65% lymphocyte proliferation activity compared to a control. Optionally the formulation retains at least 70% of T-cell activation activity compared to a reference; has a protein concentration of at least 90% of the starting concentration; has a pH of 5.5 ± 0.2.

Free of microbial contamination is meant free of mycoplasm, endotoxin and microbial (e.g., aerobic, anaerobic and fungi) contamination.

By substantially free of endotoxin is meant that there is less endotoxin per dose of than is allowed by the FDA for a biologic, which is a total endotoxin of 5 EU/kg body weight per day, which for an average 70 kg person is 350 EU per total dose

By substantially free for mycoplasma and microbial contamination is meant as negative readings for the generally accepted tests known to those skilled in the art. For example, mycoplasm contamination is determined by subculturing a formulation sample in broth medium and distributed over agar plates on day 1, 3, 7, and 14 at 37° C. with appropriate 30 positive and negative controls. The formulation sample appearance is compared microscopically, at 100x, to that of the positive and negative control. Additionally, inoculation of an indicator cell culture is incubated for 3 and 5 days and examined at 600x for the presence of mycoplasmas by epifluorescence microscopy using a DNA-binding fluorochrome. The product is considered satisfactory if the agar and/or the broth media procedure and the indicator cell culture procedure show no evidence of mycoplasma contamination. The sterility test to establish that the product is free of microbial contamination is based on the U.S. Pharmacopedia.

By substantially free of protein aggregates is meant that there is less than 5%, 4%, 3%, 2%, 1% or less protein aggregates. Aggregation is measured by any method known in the art such as UPLC.

### Therapeutic Administration

Therapeutic formulations provided herein, which include an anti-CD3 antibody formulation disclosed herein, may be used to treat or alleviate a symptom associated with an immune-related disorder, such as, for example, an autoimmune disease or an inflammatory disorder. The anti-CD3 antibody formulation disclosed herein may also be used to treat or alleviate a symptom associated with a neurodegenerative disorder or cancer.

Autoimmune diseases that may be treated by administering a formulation described herein include, for example, Acquired Immunodeficiency Syndrome (AIDS, which is a viral disease with an autoimmune component), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis herpetiformis; chronic fatigue immune dysfunction syndrome (CFIDS), chronic inflammatory demyelinating polyneuropathy (CIPD), cicatricial pemphigoid, cold agglutinin disease, crest syndrome, Crohn's disease, Degos' disease, dermatomyositis-juvenile, discoid lupus, essential mixed cryoglobulinemia, experimental autoimmune encephalomyelitis (EAE), fibromyalgia-fibromyositis, Graves' disease, Guillain-Barré syndrome, Hashimoto's thyroiditis, idiopathic pulmonary fibrosis, idiopathic thrombocytopenia purpura (ITP), IgA nephropathy, insulin-dependent diabetes mellitus (Type I diabetes; Type 2 diabetes), juvenile chronic arthritis (Still's disease), juvenile rheumatoid arthritis, Ménière's disease, mixed connective tissue disease, multiple sclerosis, myasthenia gravis, nonalcoholic steatohepatitis (NASH), pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjögren's syndrome, stiff-man syndrome, systemic lupus erythematosus, Takayasu arteritis, temporal arteritis/giant cell arteritis, ulcerative colitis, uveitis, vitiligo and Wegener's granulomatosis.

Inflammatory disorders diseases that may be treated by administering a formulation described herein include, for example, chronic and acute inflammatory disorders. Examples of inflammatory disorders include Alzheimer's disease, asthma, atopic allergy, allergy, atherosclerosis, bronchial asthma, eczema, glomerulonephritis, graft vs. host disease, hemolytic anemias, inflammatory bowel disease (IBD), nonalcoholic fatty liver disease (NAFLD), osteoarthritis, sepsis, stroke, transplantation of tissue and organs, vasculitis, diabetic retinopathy and ventilator induced lung injury.

The formulations of anti-CD3 antibody described herein may be administered to a subject suffering from an immune-related disorder, such as an autoimmune disease or an inflammatory disorder a neurodegenerative disorder or cancer. A subject suffering from an autoimmune disease, an inflammatory disorder, a neurodegenerative disorder or cancer may be identified by methods known in the art.

Administration of an anti-CD3 antibody formulation to a patient suffering from an immune-related disorder, such as an autoimmune disease, an inflammatory disorder, neurodegenerative disorder or cancer may be considered successful if any of a variety of laboratory or clinical results is achieved. For example, in some embodiments, administration of an anti-CD3 antibody formulation to a patient suffering from an immune-related disorder such as an autoimmune disease or an inflammatory disorder is considered successful if one or more of the symptoms associated with the disorder is alleviated, reduced, inhibited or does not progress to a further, *i.e.,* worse, state. In some embodiments, administration of an anti-CD3 antibody formulation to a patient suffering from an immune-related disorder such as an autoimmune disease or an inflammatory disorder is considered successful if the disorder, *e.g.,* an autoimmune disorder, enters remission or does not progress to a further, *i.e.,* worse, state.

In another embodiment, the anti-CD3 antibody formulations provided herein are used in the treatment or diagnosis of multiple sclerosis (MS). MS is a chronic, inflammatory autoimmune disease that affects the central nervous system (CNS). Symptoms of MS include, for example, changes in sensation, visual problems, muscle weakness, depression, difficulties with coordination and speech, and pain. The anti-CD3 antibody formulations provided herein may be administered to a subject that is suffering from, has been diagnosed with, is suspected of having, or is predisposed to MS. The anti-CD3 antibody formulations provided herein may be administered at a dosage that is sufficient to alleviate at least one symptom of MS, to treat MS, and/or to prevent MS from progressing to a further disease state in a subject. In some embodiments, administration of an anti-CD3 antibody formulation described herein reduces the incidence of relapse in relapse-remitting MS. In some embodiments, administration of an anti-CD3 antibody formulation described herein delays the time to relapse in relapse-remitting MS. In some embodiments, administration of an anti-CD3 antibody formulation described herein slows the progression of primary progressive MS.

In yet another embodiment, an anti-CD3 formulation provided herein is administered to a human individual to activate mucosal immunity and immunomodulation.

In some embodiments, an anti-CD3 antibody formulation provided herein is used to activate regulatory T-cells (Tregs).

In another embodiment, an anti-CD3 antibody formulation provided herein is administered to human subjects to prevent, reduce or decrease the recruitment of immune cells into human tissues. An anti-CD3 antibody used herein may be administered to a subject in need thereof to prevent and/or treat conditions associated with abnormal or deregulated immune cell recruitment into tissue sites of human disease.

In another embodiment, an anti-CD3 antibody formulation provided herein is administered to human subjects to prevent, reduce or decrease the extravasation and diapedesis of immune cells into human tissues. Thus, the anti-CD3 antibodies used herein may be administered to prevent and/or treat conditions associated with abnormal or deregulated immune cell infiltration into tissue sites of human disease.

In another embodiment, an anti-CD3 antibody formulation provided herein is administered to human subjects to prevent, reduce or decrease the effects mediated by the release of cytokines within the human body. The term "cytokine" refers to all human cytokines known within the art that bind extracellular receptors upon the cell surface and thereby modulate cell function, including but not limited to IL-2, IFN-g, TNF-a, IL-4, IL-5, IL-6, IL-9, IL-10, and IL-13.

In another embodiment, an anti-CD3 antibody formulation provided herein is administered to human subjects to prevent, reduce or decrease the effects mediated by the release of cytokine receptors within the human body. The term "cytokine receptor" refers to all human cytokine receptors within the art that bind one or more cytokine(s), as defined herein, including but not limited to receptors of the aforementioned cytokines. Thus, an anti-CD3 antibody used herein may be administered to treat and/or prevent conditions mediated through abnormal activation, binding or ligation of one or more cytokine receptor(s) within the human body. It is further envisioned that administration of the anti-CD3 antibody in vivo may deplete the intracellular signaling mediated by cytokine receptor(s) within such human subject.

In one aspect, an anti-CD3 antibody formulation provided herein is administered to a subject upon decrease of pancreatic beta-cell function. In one embodiment, the individual is tested for beta-cell function, insulin secretion or c-peptide levels using methods known in the art. Subsequently, upon determination of decrease of beta-cell function, insulin secretion or c-peptide levels, a sufficient dosage of an anti-CD3 antibody formulation provided herein is administered to the subject to prevent further progression of autoimmune destruction of beta-cell function.

### Definitions

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well-known and commonly used in the art. Standard techniques are used for recombinant DNA, oligonucleotide synthesis, and tissue culture and transformation (*e.g*., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.,* Sambrook et al. Molecular Cloning: A Laboratory Manual (2d ed., Cold Spring Harbor Laboratory Press, Cold Spring Harbor, N.Y. (1989)). The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

As used herein, the term "antibody" refers to immunoglobulin molecules and immunologically active portions of immunoglobulin (Ig) molecules, *i*.*e*., molecules that contain an antigen binding site that specifically binds (immunoreacts with) an antigen. Such antibodies include, but are not limited to, polyclonal, monoclonal, chimeric, single chain, F_{ab}, F_{ab'} and F_{(ab')2} fragments, and an F_{ab} expression library. By "specifically bind" or "immunoreacts with" is meant that the antibody reacts with one or more antigenic determinants of the desired antigen and does not react (i.e., bind) with other polypeptides or binds at much lower affinity (K_{d} > 10⁻⁶) with other polypeptides.

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgA, and IgE, respectively. Within light and heavy chains, the variable and constant regions are joined by a "J" region of about 12 or more amino acids, with the heavy chain also including a "D" region of about 10 more amino acids. *See* generally, Fundamental Immunology Ch. 7 (Paul, W., ea., 2nd ed. Raven Press, N.Y. (1989)). The variable regions of each light/heavy chain pair form the antibody binding site.

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs contain an antigen binding site capable of immunoreacting with a particular epitope of the antigen characterized by a unique binding affinity for it.

In general, antibody molecules obtained from humans relate to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain.

As used herein, the term "epitope" includes any protein determinant capable of specific binding to an immunoglobulin, a scFv, or a T-cell receptor. The term "epitope" includes any protein determinant capable of specific binding to an immunoglobulin or T-cell receptor. Epitopic determinants usually consist of chemically active surface groupings of molecules such as amino acids or sugar side chains and usually have specific three-dimensional structural characteristics, as well as specific charge characteristics. An antibody is said to specifically bind an antigen when the dissociation constant is ≤ 1 µM; preferably ≤ 100 nM and most preferably ≤ 10 nM.

As used herein, the terms "immunological binding" and "immunological binding properties" and "specific binding" refer to the non-covalent interactions of the type which occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides are quantified using methods well known in the art. One such method entails measuring the rates of antigen-binding site/antigen complex formation and dissociation, wherein those rates depend on the concentrations of the complex partners, the affinity of the interaction, and geometric parameters that equally influence the rate in both directions. Thus, both the "on rate constant" (Kₒₙ) and the "off rate constant" (K_{off}) can be determined by calculation of the concentrations and the actual rates of association and dissociation. (*See* Nature 361:186-87 (1993)). The ratio of K_{off}/Kₒₙ enables the cancellation of all parameters not related to affinity, and is equal to the dissociation constant K_{d}. (*See, generally,* Davies et al. (1990) Annual Rev Biochem 59:439-473). An antibody of the present invention is said to specifically bind to a CD3 epitope when the equilibrium binding constant (K_{d}) is ≤1 µM, preferably ≤ 100 nM, more preferably ≤ 10 nM, and most preferably ≤ 100 pM to about 1 pM, as measured by assays such as radioligand binding assays or similar assays known to those skilled in the art.

Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide- containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur- containing side chains is cysteine and methionine. Preferred conservative amino acids substitution groups are: valine-leucine-isoleucine, phenylalanine-tyrosine, lysine-arginine, alanine valine, glutamic- aspartic, and asparagine-glutamine.

As discussed herein, minor variations in the amino acid sequences of antibodies or immunoglobulin molecules are contemplated as being encompassed by the present invention, providing that the variations in the amino acid sequence maintain at least 75%, more preferably at least 80%, 90%, 95%, and most preferably 99%. In particular, conservative amino acid replacements are contemplated. Conservative replacements are those that take place within a family of amino acids that are related in their side chains. Genetically encoded amino acids are generally divided into families: (1) acidic amino acids are aspartate, glutamate; (2) basic amino acids are lysine, arginine, histidine; (3) non-polar amino acids are alanine, valine, leucine, isoleucine, proline, phenylalanine, methionine, tryptophan, and (4) uncharged polar amino acids are glycine, asparagine, glutamine, cysteine, serine, threonine, tyrosine. The hydrophilic amino acids include arginine, asparagine, aspartate, glutamine, glutamate, histidine, lysine, serine, and threonine. The hydrophobic amino acids include alanine, cysteine, isoleucine, leucine, methionine, phenylalanine, proline, tryptophan, tyrosine and valine. Other families of amino acids include (i) serine and threonine, which are the aliphatic-hydroxy family; (ii) asparagine and glutamine, which are the amide containing family; (iii) alanine, valine, leucine and isoleucine, which are the aliphatic family; and (iv) phenylalanine, tryptophan, and tyrosine, which are the aromatic family.

The term "agent" is used herein to denote a chemical compound, a mixture of chemical compounds, a biological macromolecule, or an extract made from biological materials.

The term patient includes human and veterinary subjects. The terms "subject" and "patient" are used interchangeably herein.

The disclosure also includes Fᵥ, F_{ab}, F_{ab'} and F_{(ab')2} anti-CD3 antibody fragments, single chain anti-CD3 antibodies, bispecific anti-CD3 antibodies, heteroconjugate anti-CD3 antibodies, trispecific antibodies, immunoconjugates and fragments thereof.

Bispecific antibodies are antibodies that have binding specificities for at least two different antigens. In the present case, one of the binding specificities is for CD3. The second binding target is any other antigen, and advantageously is a cell-surface protein or receptor or receptor subunit.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The disclosure having now been described by way of written description, those of skill in the art will recognize that the disclosure can be practiced in a variety of embodiments and that the foregoing description and examples below are for purposes of illustration and not limitation of the claims that follow.

### Other Embodiments

While the disclosure has been described in conjunction with the detailed description thereof, the foregoing description is intended to illustrate and not limit the scope of the disclosure, which is defined by the scope of the appended claims. Other aspects, advantages, and modifications are within the scope of the following claims.

### EXAMPLES

The examples described in this section are provided for illustration only and are not intended to limit the invention in any way.

### Example 1: Evaluation of Foralumab metered dose delivery using an Aptar Device

Foralumab Drug Product was formulated to the intended dosage concentration by dilution with Acetate Buffered Saline, pH 5.5.

The Formulation is as follows:

**Table 1: Formulation**

| **Description** | **0004-047A 25µg/100µl (mg/mL)** | **0004-048A 50µg/100µl (mg/mL)** |
|---|---|---|
| Foralumab | 0.25 | 0.50 |
| Sodium Acetate, Trihydrate, USP | 3.40 | 3.40 |
| Polysorbate 80, USP | 0.20 | 0.20 |
| Sodium Chloride, USP | 7.31 | 7.31 |
| Purified Water, USP | 988.84 | 988.59 |
| **Total** | **1000 mg** | **1000 mg** |

The Aptar UniDose device is prefilled with the formulation. Intranasal administration is achieved by manually depressing the bottom of the unit directly through one nostril. The process is repeated for the second nostril.

The purpose of the investigations described in this document was to record those attributes of the nasal products that are deemed to be important for nasal delivery of Foralumab:
1) Delivered dose
2) Droplet size distribution of the delivered dose.
3) Spray pattern
4) Plume Geometry

Due to the limited number of available samples, only the Drug Product is evaluated. The Placebo will be evaluated at a separate time.

### Dose Content Uniformity

Dose Content Uniformity (Shot weight) is used to determine the reproducibility of the individual sprays from the individual devices. The unit is weighed. The unit is manually actuated for a single dose. The unit is reweighed to determine the amount of liquid that was dispensed in milligrams. The procedure is then repeated for a total of 10 separate devices. The results are shown in Table 2:

**Table 2: Dose delivered per actuation (as weight loss from the device/actuation)**

| **Device** | **25µg/100µl 0004-047A (mg)** | **50µg/100µl 0004-048A (mg)** |
|---|---|---|
| 1 | 102 | 103 |
| 2 | 99 | 101 |
| 3 | 102 | 95 |
| 4 | 97 | 102 |
| 5 | 101 | 104 |
| 6 | 102 | 89 |
| 7 | 102 | 103 |
| 8 | 101 | 102 |
| 9 | 102 | 103 |
| 10 | 97 | 104 |
| Mean | 100 | 100.6 |
| RSD | 2.0% | 4.6% |
| Minimum | 97 | 89 |
| Maximum | 102 | 104 |

The results show that the delivered dose is uniform as shown by the %RSD. These results are within the USP requirements for Dose Content Uniformity. The delivered dose of the drug product will be tested using a drug specific assay if this device is selected for further study.

### Droplet Size Distribution

The droplet size was measured using laser diffraction. A Malvern Spraytec unit was used in the analysis. Five (5) separate devices were placed one inch below the laser beam and manually sprayed into the Malvern and the results were averaged.

The Malvern Parameters are as follows:

| | |
|---|---|
| Measurement Values and Settings Instrument = Spraytec - Open Spray | |
| Lens = 300mm | Path Length = 12.0 (mm) |
| Particulate Refractive Index = 1.33 + 0.000i | Scatter start = 9 Dispersant |
| Refractive Index = 1.00 | Scatter end = 36 |
| Particle Density = 1.00 (gm/cc) | Scattering threshold = 1 |
| Residual = 0.36 (%) | Minimum size = 0.10 (µm) |
| Extinction analysis = Off | Maximum size = 900.00 (µm) |
| Actuator distance = 0 | |
| Multiple Scatter = Off Actuator angle = 0 Software = v3.30.016 | |
| Serial Numbers: Instrument = MAL1225354 | |
| Detector = 08CX-CDL | |
| SOP Name = Nasal Spray [2].ssop | |

The results are shown in Table 3 and Table 4:

**Table 3: 25µg/100µl 0004-047A**

| **Device** | **Dx(10)** | **Dx(50)** | **Dx(90)** |
|---|---|---|---|
| | **µm (Volume Based)** | **µm (Volume Based)** | **µm (Volume Based)** |
| 1 | 18.30 | 35.06 | 68.90 |
| 2 | 17.33 | 38.87 | 82.07 |
| 3 | 17.80 | 33.60 | 60.96 |
| 4 | 17.32 | 33.48 | 62.34 |
| 5 | 17.08 | 35.46 | 70.18 |
| 6 | 17.26 | 35.80 | 71.59 |
| 7 | 16.87 | 37.81 | 81.61 |
| 8 | 16.94 | 33.47 | 63.73 |
| 9 | 16.05 | 33.63 | 68.40 |
| 10 | 16.79 | 34.98 | 69.18 |
| Average | 17.17 | 35.22 | 69.90 |

**Table 4: 50µg /100µl 0004-048A**

| **Device** | **Dx(10)** | **Dx(50)** | **Dx(90)** |
|---|---|---|---|
| | **µm (Volume Based)** | **µm (Volume Based)** | **µm (Volume Based)** |
| 1 | 17.50 | 32.59 | 58.67 |
| 2 | 17.68 | 33.88 | 61.60 |
| 3 | 20.93 | 36.71 | 64.61 |
| 4 | 18.64 | 36.11 | 67.24 |
| 5 | 16.27 | 35.37 | 74.57 |
| 6 | 16.84 | 37.11 | 79.17 |
| 7 | 17.19 | 34.79 | 71.59 |
| 8 | 18.51 | 38.06 | 76.65 |
| 9 | 16.94 | 41.80 | 100.87 |
| 10 | 17.40 | 42.83 | 111.58 |
| Average | 17.79 | 36.93 | 76.66 |

The above results are very consistent and reproducible.

### Spray Pattern

The spray pattern is a cross-sectional representation of the plume upon actuation. A TLC plate was suspended 3 cm above the nosepiece and the device was actuated. The maximum diameter, minimum diameter and shape of the pattern is recorded in centimeters. The results are shown in Table 5 and Table 6:

**Table 5: 25µg/100µl 0004-047**

| **Syringe** | **D_{Max} (cm)** | **Dₘᵢₙ (cm)** | **Shape** |
|---|---|---|---|
| 1 | 3.5 | 3.0 | Circular |
| 2 | 3.4 | 3.4 | Circular |
| 3 | 3.4 | 3.4 | Circular |
| 4 | 3.4 | 2.9 | Circular |
| 5 | 3.0 | 2.8 | Circular |
| Average | 3.3 | 3.1 | N/A |

**Table 6: 50µg/100µl 0004-048**

| **Syringe** | **D_{Max} (cm)** | **Dₘᵢₙ (cm)** | **Shape** |
|---|---|---|---|
| 1 | 3.1 | 3.0 | Circular |
| 2 | 3.7 | 3.3 | Circular |
| 3 | 3.2 | 3.1 | Circular |
| 4 | 3.3 | 3.0 | Circular |
| 5 | 3.0 | 2.9 | Circular |
| Average | 3.3 | 3.1 | N/A |

### Plume Geometry

The plume geometry measures the angle of the plume. As the device was actuated a photograph of the plume was taken. The edges of the plume were determined, and a straight line was drawn until both sides of the plume intersect. The angle generated is recorded in Table 7and Table 8 below:

**Table 7: 25µg/100µl 0004-047**

| **Device** | **Plume Angle** |
|---|---|
| 1 | 53° |
| 2 | 42° |
| 3 | 53° |
| 4 | 45° |
| 5 | 45° |
| Average | 48° |

**Table 8: 50µg/100µl 0004-048**

| **Device** | **Plume Angle** |
|---|---|
| 1 | 49° |
| 2 | 55° |
| 3 | 46° |
| 4 | 52° |
| 5 | 48° |
| Average | 50° |

### Example 2: Evaluation of Foralumab metered dose delivery using the Gerresheimer Device

A unit dose device was developed. The device consists of 2 separate parts:

1mL Type I, glass syringe equipped with a plunger - Manufacturer Gerresheimer. This item is described in a Gerresheimer DMF filed with the FDA.
- Nasal Atomization Device (NAD 300)- Manufacturer Teleflex. This item is described in a Teleflex DMF filed with the FDA.

Foralumab Drug Product is formulated to the intended dosage concentration by dilution with Acetate Buffered Saline, pH 5.5, and prefilled into a Type I glass Gerresheimer Syringe. The Formulation is as follows:

**Table 9: Formulation**

| **Description** | **0004-047 Placebo (mg/mL)** | **0004-047G 25µg/100µl (mg/mL)** | **0004-048G 50µg/100µl (mg/mL)** |
|---|---|---|---|
| Foralumab | 0.00 | 0.25 | 0.50 |
| Sodium Acetate, Trihydrate, USP | 3.40 | 3.40 | 3.40 |
| Polysorbate 80, USP | 0.20 | 0.20 | 0.20 |
| Sodium Chloride, USP | 7.31 | 7.31 | 7.31 |
| Purified Water, USP | 989.09 | 988.84 | 988.59 |
| **Total** | **1000 mg** | **1000 mg** | **1000 mg** |

Intranasal administration is achieved by attaching a Teleflex Nasal Atomization Device to the filled syringe (Gerresheimer Device). The dose is then administered directly through one nostril. The process is repeated for the second nostril.

The purpose of the investigations described in this document was to record those attributes of the nasal products that are deemed to be important for nasal delivery of Foralumab:
1) Delivered dose
2) Droplet size distribution of the delivered dose.
3) Spray pattern
4) Plume Geometry

### Dose Content Uniformity

Dose Content Uniformity (Shot weight) is used to determine the reproducibility of the individual sprays from the individual syringes. The unit is weighed. The unit is manually actuated for a single dose. The unit is reweighed to determine the amount of liquid that was dispensed in milligrams. The procedure is then repeated for a total of 10 separate syringes connected to 10 separate atomizers. The results are shown in Table 10:

**Table 10: Dose delivered per actuation**

| **Device** | **Placebo 0004-047 (mg)** | **25µg/100µl 0004-047G (mg)** | **50µg/100µl 0004-048G (mg)** |
|---|---|---|---|
| 1 | 99 | 128 | 117 |
| 2 | 109 | 107 | 115 |
| 3 | 101 | 137 | 107 |
| 4 | 105 | 130 | 119 |
| 5 | 93 | 110 | 118 |
| 6 | 108 | 124 | 110 |
| 7 | 104 | 113 | 118 |
| 8 | 97 | 106 | 116 |
| 9 | 107 | 121 | 97 |
| 10 | 109 | 118 | 110 |
| Mean | 103.2 | 119.4 | 112.7 |
| RSD | 5.1% | 8.3% | 5.8% |
| Minimum | 93 | 106 | 97 |
| Maximum | 109 | 137 | 119 |

The results show that the delivered dose is uniform as shown by the %RSD. These results are within the USP requirements for Dose Content Uniformity. The delivered dose of the drug product will be tested using a drug specific assay if this device is selected for further study.

### Droplet Size Distribution

The droplet size was measured using laser diffraction. A Malvern Spraytec unit was used in the analysis. Ten (10) separate syringes equipped with ten (10) separate Nasal Atomization Devices were placed one inch below the laser beam and manually sprayed into the Malvern and the results were averaged.

The Malvern Parameters are as follows:
Measurement Values and Settings
- Instrument = Spraytec - Open Spray
- Lens = 300mm
- Path Length = 12.0 (mm)
- Particulate Refractive Index = 1.33 + 0.000i
- Scatter start = 9
- Dispersant Refractive Index = 1.00
- Scatter end = 36
- Particle Density = 1.00 (gm/cc)
- Scattering threshold = 1
- Residual = 0.36 (%)
- Minimum size = 0.10 (µm)
- Extinction analysis = Off
- Maximum size = 900.00 (µm)
- Actuator distance = 0
- Multiple Scatter = Off
- Actuator angle = 0 Software = v3.30.016
- Serial Numbers: I
- Instrument = MAL1225354
- Detector = 08CX-CDL
- SOP Name = Nasal Spray [2].ssop

The results are shown in Table 11, Table 12 and Table 13 below:

**Table 11: Placebo 0004-047**

| **Device** | **Dx(10) µm (Volume Based)** | **Dx(50) µm (Volume Based)** | **Dx(90) µm (Volume Based)** |
|---|---|---|---|
| 1 | 26.34 | 85.69 | 228.70 |
| 2 | 35.67 | 111.21 | 250.37 |
| 3 | 27.71 | 87.58 | 225.69 |
| 4 | 23.04 | 55.51 | 173.54 |
| 5 | 31.48 | 89.18 | 226.37 |
| 6 | 24.16 | 62.64 | 209.02 |
| 7 | 36.78 | 113.32 | 251.73 |
| 8 | 50.64 | 136.00 | 268.91 |
| 9 | 37.32 | 111.40 | 249.41 |
| 1 0 | 32.06 | 102.49 | 243.07 |
| Average | 32.52 | 95.50 | 232.68 |

**Table 12: 25µg/100µl 0004-047G**

| **Device** | **Dx(10) µm (Volume Based)** | **Dx(50) µm (Volume Based)** | **Dx(90) µm (Volume Based)** |
|---|---|---|---|
| 1 | 20.58 | 58.14 | 157.12 |
| 2 | 18.20 | 42.84 | 94.44 |
| 3 | 35.32 | 103.12 | 238.81 |
| 4 | 31.30 | 80.29 | 196.84 |
| 5 | 16.85 | 41.08 | 97.27 |
| 6 | 56.01 | 132.19 | 264.74 |
| 7 | 37.11 | 104.76 | 240.50 |
| 8 | 23.48 | 58.32 | 137.42 |
| 9 | 33.41 | 98.09 | 233.92 |
| 1 0 | 15.98 | 33.03 | 69.01 |
| Average | 28.82 | 75.19 | 173.01 |

**Table 13: 50 µg /100µl 0004-048G**

| **Device** | **Dx(10) µm (Volume Based)** | **Dx(50) µm (Volume Based)** | **Dx(90) µm (Volume Based)** |
|---|---|---|---|
| 1 | 21.08 | 52.55 | 129.88 |
| 2 | 20.43 | 50.14 | 122.97 |
| 3 | 19.62 | 47.88 | 111.34 |
| 4 | 32.00 | 100.87 | 237.96 |
| 5 | 21.15 | 46.62 | 99.45 |
| 6 | 42.99 | 119.51 | 255.19 |
| 7 | 23.12 | 65.52 | 172.80 |
| 8 | 17.29 | 41.74 | 103.50 |
| 9 | 22.01 | 61.13 | 161.44 |
| 1 0 | 25.54 | 78.15 | 206.59 |
| Average | 24.52 | 66.41 | 160.11 |

There seems to be variability between devices. This is most likely due to the design and limitations of the device.

### Spray Pattern

The spray pattern is a cross-sectional representation of the plume upon actuation. A TLC plate was suspended 3 cm above the nosepiece and the device was manually actuated. The maximum diameter, minimum diameter and shape of the pattern is recorded in centimeters. The results are shown in Table 14, Table 15 and Table 16:

**Table 14: Placebo 0004-047**

| **Device** | **D_{Max} (cm)** | **Dₘᵢₙ (cm)** | **Shape** |
|---|---|---|---|
| 1 | 2.7 | 2.5 | Circular |
| 2 | 2.7 | 2.5 | Circular |
| 3 | 2.2 | 2.2 | Circular |
| 4 | 3.2 | 2.2 | Oval |
| 5 | 2.6 | 1.8 | Oval |
| 6 | 3.1 | 1.4 | Oval |
| 7 | 2.7 | 2.3 | Circular |
| 8 | 2.9 | 2.6 | Circular |
| 9 | 2.7 | 2.6 | Circular |
| 10 | 2.4 | 2.3 | Circular |
| Average | 2.7 | 2.2 | N/A |

**Table 15: 25µg/100µl 0004-047G**

| **Syrin ge** | **D_{Max} (cm)** | **Dₘᵢₙ (cm)** | **Shape** |
|---|---|---|---|
| 1 | 2.8 | 2.5 | Circular |
| 2 | 3.0 | 1.7 | Oval |
| 3 | 3.0 | 1.2 | Oval |
| 4 | 2.2 | 1.7 | Circular |
| 5 | 2.5 | 2.5 | Circular |
| Avera ge | 2.7 | 1.9 | N/A |

**Table 16: 50µg/100µl 0004-048G**

| **Syrin ge** | **D_{Max} (cm)** | **Dₘᵢₙ (cm)** | **Shape** |
|---|---|---|---|
| 1 | 2.1 | 2.2 | Circular |
| 2 | 2.2 | 2.0 | Circular |
| 3 | 3.0 | 2.2 | Circular |
| 4 | 3.1 | 1.5 | Oval |
| 5 | 2.8 | 2.5 | Circular |
| Avera ge | 2.6 | 2.1 | N/A |

There seems to be variability between devices. This is most likely due to the design and limitations of the device.

### Plume Geometry

The plume geometry measures the angle of the plume. As the device was actuated a photograph of the plume was taken. The edges of the plume were determined, and a straight line was drawn until both sides of the plume intersect. The angle generated is recorded in Table 17, Table 18 and Table 19 below:

**Table 17: Placebo 0004-047**

| **Device** | **Plume Angle** |
|---|---|
| 1 | 14° |
| 2 | 18° |
| 3 | 15° |
| 4 | 17° |
| 5 | 17° |
| 6 | 11° |
| 7 | 19° |
| 8 | 15° |
| 9 | 19° |
| 10 | 23° |
| Average | 17° |

**Table 18: 25µg/100µl 0004-047G**

| **Device** | **Plume Angle** |
|---|---|
| 1 | 16° |
| 2 | 19° |
| 3 | 26° |
| 4 | 38° |
| 5 | 26° |
| Average | 25° |

**Table 19: 50µg/100µl 0004-048G**

| **Device** | **Plume Angle** |
|---|---|
| 1 | 24° |
| 2 | 38° |
| 3 | 15° |
| 4 | 19° |
| 5 | 23° |
| Average | 24° |

### Example 3: Stability of Nasal Foralumab Formulation

The stability of a nasal formulation comprising 25 µg or 50 µg foralumab in, 3.4 mg/mL sodium acetate, 0.20 mg/ml polysorbate 80 and 7.31 mg/ ml sodium chloride in a total volume of 100 µL was analyzed. Stability was determined after storage at about 5°C (2°C to 8°C) for 1 months, 3 months, 6 months or 12 months. Stability was evaluated by the following read outs: appearance, protein concentration, impurities, pH, capillary isoelectric focusing (cIEF), bioburden, and potency. Appearance-visual; protein concentration -UV absorbance at 280 nm; assay and impurities by reversed phase UPLC; cIEF- capillary isoelectric focusing; pH-potentiometric; bioburden-USP<61,62>; potency by either T cell proliferation assay or NAFT activation in pan T cells

Results are shown in Table 20, Table 21, Table 22, and Table 23.

Actuated data-samples are collected after spraying from the Aptar device and tested for any effects of shear from spraying are impactful on critical quality attributes. Unactuated data-samples are collected directly from the type1 glass/Aptar plunger container closure system without spraying so no stress.

**Table 20: Stability of Nasal Foralumab Formulation (25 µg/100 uL) at 5°C (2-8°C) (Manufacturing Date: 23Sep2021, Stability Start Date: 23Nov2021)**

| **Testing** | **Method** | **Specification** | **Initial** | **1M** | **3M** | **6M** | **12M** |
|---|---|---|---|---|---|---|---|
| | | **Pull Date** | **N/A** | **27Dec21** | **23Feb22** | **23May22** | **23Nov2022** |
| **Appearance** | ATM-1095 | Clear, colorless to slightly yellow liquid, free from visible particles | Conform | Conform | Conform | Conform | Conform |
| **Assay Protein** | ATM-1862 | 90.0%-110.0% of label concentration | 101.2% | 101.5% | 103.5% | 103.5% | 100.7% |
| **Impurities** | ATM-1862 | Aggregates NMT 3% | 0% (0.4%) | 0% (0.48%) | 1% (0.55%) | 1% (0.63%) | 1% (0.52%) |
| **pH** | USP<791> | 5.5 ± 0.2 | 5.6 | 5.6 | 5.7 | 5.7 | 5.6 |
| **Capillary Isoelectric Focusing (cIEF)** | ATM-2263 | pI of main peak conforms to reference standard. Report. pI values of individual peaks in test sample. | pI of main peak conforms to reference standard¹ | pI of main peak conforms to reference standard¹ | pI of main peak conforms to reference standard¹ | pI of main peak conforms to reference standard¹ | pI of main peak conforms to reference standard¹ |
| | | | Peak1: 8.90, Peak2: 9.19, Peak3: 9.24, Peak4 (main): 9.28, Peak5:9.43 | Peak1: 8.92, Peak2: 9.18, Peak3: 9.23, Peak4 (main): 9.27, Peak5:9.37 | Peak1: 8.91, Peak2: 9.17, Peak3: 9.22, Peak4 (main) 9 26, Peak5:9.39 | Peak1: 8.90, Peak2: 9.18, Peak3: 9.23, Peak4 (main)⁻9.27, Peak5:9.39 | Peak1: 8.91, Peak2: 9.18, Peak3: 9.23, Peak4 (main) 9.27, Peak5:9.41 |
| **Bioburden** | USP<61> | Total Aerobic Microbial Count: NMT 100 cfu/g Total Yeasts and Mold Count: NMT 10 cfu/g S. aureus: Absent/1g Ps. Aerugosina: Absent/1g | | | | Total Aerobic Microbial Count: <10 cfu/g Total Yeasts and Mold Count: <10 cfu/g S. aureus: Absent/1g Ps. Aerugosina: Absent/1g | Pending |
| | USP<62> | | | | | | |
| **Potency** | Lymphocyte proliferation assay | 65%-100% activity relative to control | | | | Not tested | |
| | T cell activation | 70%-130% of reference | | | | | 90% |
| **Reference** | | | NB3284, 3285, 3349 | NB3285, 3348 | NB3439, 3158, 3348 | NB3510, 3532, 3535 Nelson Lab Report# R-619817-R1 | NB3535, 3626, 3638 BioAgilytix Record# CT08DEC22I TH |

| | | | | | | | |
|---|---|---|---|---|---|---|---|
| 1. Sample's main peak pI value is within ± 0.10 of mean pI value of main peak of reference standard. Sample electrophoretic profile is comparable to the electrophoretic profile of reference standard. | | | | | | | |

**Table 21: Stability of Nasal Foralumab Formulation (25 µg/100 uL) at 5°C (2-8°C) (Manufacturing Date: 24Mar2021, Stability Start Date: 22Dec2021)**

| **Testing** | **Method** | **Specification** | **Initial** | **1M** | **3M** | **6M** | **12M** |
|---|---|---|---|---|---|---|---|
| | | **Pull Date** | **N/A** | **24Jan2022** | **23Mar2022** | **22Jun2022** | **21Dec2022** |
| Appearance | ATM-1095 | Clear, colorless to slightly yellow | Conform | Conform | Conform | Conform | Conform |
| | | liquid, free from visible particles | | | | | |
| Assay (Protein Concentration) | ATM-1862 | 90.0 % - 110.0% of label concentration | 103.1% | 107.9% | 104.6% | 104.6% | 99.3% |
| Impurities | ATM-1862 | Aggregates NMT 3% | 1% (0.71%) | 1% (0.82%) | 1% (0.37%) | 1% (0.60%) | 0% (0.49%) |
| pH | USP<791> | 5.5 ± 0.2 | 5.5 | 5.5 | 5.6 | 5.6 | 5.5 |
| Capillary Isoelectric Focusing (cIEF) | ATM-2263 | pI of main peak conforms to reference standard. | pI of main peak conforms to reference standard¹ | pI of main peak conforms to reference standard¹ | pI of main peak conforms to reference standard¹ | pI of main peak conforms to reference standard | |
| | | | | | | Peak1: 8.90, Peak2: 9.17, Peak3: 9.22, Peak 4 (main): 9.26, Peak5: 9.39 | pI of main peak conforms to reference standard¹ |
| | | | Peak1: 8.90, Peak2: 9.19, Peak3: 9.24, Peak4 (main): 9.28, Peak5: 9.44 | Peak1: 8.90, Peak2: 9.18, Peak3: 9.23, Peak4 (main): 9.26, Peak5: 9.35 | Peak1: 8.92, Peak2: 9.18, Peak3: 9.23, Peak4 (main); 9.27, Peak5: 9.37 | | Peak1: 8.90, Peak2: 9.18, Peak3: 9.22, Peak4 (main); 9.26, Peak5: 9.40 |
| | | Report pI values of individual peaks in test sample | | | | | |
| | | | | | | **02LINV-12:** pI of main peak conforms to reference standard¹ | |
| | | | | | | ①Peak1: 8.91, Peak2: 9.18, Peak3: 9.23, Peak4 (main): | |
| | | | | | | 9.27, Peak 5: 9.41 | |
| | | | | | | ②Peak1: 8.90, Peak2: 9.18, Peak3: 9.23, Peak4 (main): 9.27, Peak5: 9.42 | |
| | | | | | | ③Peak1: 8.91, Peak2: 9.18, Peak3: 9.23, Peak4 (main): 9.27, Peak5: 9.40 | |
| Bioburden | USP<61> | Total Aerobic Microbial Count: NMT 100 cfu/g | | | | Total Aerobic Microbial Count: < 10 cfu/g | Total Aerobic Microbial Count: < 10 cfu/g |
| | | Total Yeasts and Mold Count: NMT 10 cfu/g | | | | | |
| | | | | | | Total Yeasts and Mold | Total Yeasts and Mold |
| | USP<62> | S. aureus: Absent/ 1g | | | | Count: < 10 cfu/g | Count: < 10 cfu/g |
| | | Ps. Aerugosina: Absent/ 1g | | | | S. aureus: Absent/ 1g | S. aureus: Absent/ 1g |
| | | | | | | Ps. Aerugosina: Absent/ 1g | Ps. Aerugosina: Absent/ 1g |
| Potency | Lymphocyte proliferation assay | 65% - 100% activity relative to control | | | | 85.9% | |
| | T cell activation assay | 70%-130% of reference product | | | | | 97% |
| Reference | | | NB3285, 3348 | NB3377, 3348 | NB3158, 3439 | NB3480, 3510, 3532, 3535, Nelson Lab Report# R-620599-R0 Lab Form# BWH-001, Report Date: 13Sep22 | NB3638, 3535, 3626 Nelson Lab Report# R-625768-R0 BioAgilytix Record # CT03FEB23SK P01 |

| | | | | | | | |
|---|---|---|---|---|---|---|---|
| 1. Sample's main peak pI value is within ± 0.10 of mean pI value of main peak of reference standard. Sample electrophoretic profile is comparable to the electrophoretic profile of reference standard. | | | | | | | |

**Table 22: Stability of Nasal Foralumab Formulation (50 µg/100 uL) at 5°C (2-8°C) (Manufacturing Date: 27Aug2021, Stability Start Date: 23Nov2021), actuated data**

| **Testing** | **Method** | **Specification** | **Initial** | **1M** | **3M** | **6M** | **12M** |
|---|---|---|---|---|---|---|---|
| | | **Pull Date** | **N/A** | **27Dec21** | **23Feb22** | **23May22** | **23Nov2022** |
| Appearance | ATM-1095 | Clear, colorless to slightly yellow liquid, free from visible particles | Conform | Conform | Conform | Conform | Conform |
| pH | USP<791> | 5.5 ± 0.5 | 5.6 | 5.6 | 5.8 | 5.7 | 5.7 |
| Assay Protein | ATM-1862 | 90.0%-110.0% of Label claim | 99.3% | 102.0% | 101.4% | 100.8% | 98.5% |
| Impurities | ATM-1862 | Aggregates NMT 3% | 1% (0.68%) | 1% (0.72%) | 1% (0.67%) | 1% (0.62%) | 0% (0.39%) |
| Capillary Isoelectric Focusing (cIEF) | ATM-2263 | pI of main peak conforms to reference standard. Report. pI values of individual peaks in test sample. | pI of main peak conforms to reference standard¹ Peak1: | pI of main peak conforms to reference standard² | pI of main peak conforms to reference standard¹ | pI of main peak conforms to reference standard¹ | pI of main peak conforms to reference standard¹ |
| | | | 8.89, Peak2: 9.19, Peak3: 9.24, Peak4 (main): 9.28, Peak5:9.41 | Peak1: 8.91, Peak2: 9.17, Peak3: 9.22, Peak4 (main): 9.26, Peak5:9.36 | Peak1: 8.91, Peak2: 9.18, Peak3: 9.22, Peak4 (main) 9 26, Peak5:9.35 | Peak1: 8.91, Peak2: 9.20, Peak3: 9.24, Peak4 (main) 9.27, Peak5:9.41 | Peak1: 8.90, Peak2: 9.18, Peak3: 9.23, Peak4 (main) 9.26, Peak5:9.42 |
| Bioburden | USP<61> | Total Aerobic Microbial Count: NMT 100 cfu/g | | | | Total Aerobic Microbial Count: <10 cfu/g | Pending |
| | | | | | | Total Yeasts and Mold Count: <10 cfu/g | |
| | | Total Yeasts and Mold Count: NMT 10 cfu/g | | | | | |
| | USP<62> | | | | | | |
| | | S. aureus: Absent/1g | | | | S. aureus: Absent/1g Ps. | |
| | | Ps. Aerugosina: Absent/1g | | | | Aerugosina: Absent/1g | |
| Potency | Lymphocyte proliferation assay | 65%-100% activity relative to control | | | | Not tested | |
| | T cell activation | 70%-130% of reference | | | | | 79% |
| Reference | | | NB3285, 3349 | NB3285, 3348 | NB3439, 3158, 3348 | NB3532, 3535, 3510 Nelson Lab Report# R-619819-R1 | NB3535, 3626, 3638 BioAgilytix Record# CT08DEC22MK B02 |

| | | | | | | | |
|---|---|---|---|---|---|---|---|
| 1. Sample's main peak pI value is within ± 0.10 of mean pI value of main peak of reference standard. Sample electrophoretic profile is comparable to the electrophoretic profile of reference standard. | | | | | | | |

**Table 23 Stability of Nasal Foralumab Formulation (50 µg/100 uL) at 5°C (2-8°C) (Manufacturing Date: 27Aug2021, Stability Start Date: 23Nov2021), non-actuated data**

| **Testing** | **Method** | **Specification** | **Initial** | **1M** | **3M** | **6M** | **12M** |
|---|---|---|---|---|---|---|---|
| | | **Pull Date** | **N/A** | **27Dec21** | **23Feb22** | **23May22** | **23Nov2022** |
| Appearance | ATM-1095 | Clear, colorless to slightly yellow liquid, free from visible particles | Conform | Conform | Conform | Conform | Conform |
| pH | USP<791> | 5.5 ± 0.5 | 5.6 | 5.6 | 5.7 | 5.7 | 5.7 |
| Assay Protein | ATM-1862 | 90.0%-110.0% of Label claim | 100.3% | 101.3% | 101.1% | 99.7% | 98.8% |
| Impurities | ATM-1862 | Aggregates NMT 3% | 1% (0.67%) | 1% (0.71%) | 1% (0.68%) | 1% (0.61%) | 0% (0.40%) |
| Capillary Isoelectric Focusing (cIEF) | ATM-2263 | pI of main peak conforms to reference standard. Report. pI values of individual peaks in test sample. | pI of main peak conforms to reference standard** | pI of main peak conforms to reference standard¹ | pI of main peak conforms to reference standard¹ | pI of main peak conforms to reference standard¹ | pI of main peak conforms to reference standard¹ |
| | | | Peak1: 8.90, Peak2: 9.19, Peak3: 9.24, Peak4 (main): 9.28, Peak5:9.42 | Peak1: 8.91, Peak2: 9.18, Peak3: 9.23, Peak4 (main): 9.27, Peak5:9.36 | Peak1: 8.90, Peak2: 9.18, Peak3: 9.22, Peak4 (main) 9 26, Peak5:9.38 | Peak1: 8.90, Peak2: 9.19, Peak3: 9.23, Peak4 (main)⁻9.27, Peak5:9.39 | Peak1: 8.91, Peak2: 9.18, Peak3: 9.23, Peak4 (main) 9.27, Peak5:9.44 |
| Bioburden | | Total Aerobic Microbial Count: NMT 100 cfu/g | | | | Total Aerobic Microbial Count: <10 cfu/g | Pending |
| | USP<61> | Total Yeasts and Mold Count: NMT 10 cfu/g | | | | Total Yeasts and Mold Count: <10 cfu/g | |
| | USP<62> | | | | | | |
| | | S. aureus: Absent/1g Ps. Aerugosina: Absent/1g | | | | S. aureus: Absent/1g Ps. Aerugosina: Absent/1g | |
| Potency | Lymphocyte proliferation assay | 65%-100% activity relative to control | | | | Not tested | |
| | T cell activation | 70%-130% of reference | | | | | 78% |
| Reference | | | NB3285, 3349 | NB3285, 3348 | NB3439, 3158, 3348 | NB3532, 3535, 3510 Nelson Lab Report# R-619819-R1 | NB3535, 3626, 3638 BioAgilytix Record# CT08DEC22MK B02 |

| | | | | | | | |
|---|---|---|---|---|---|---|---|
| 1. Sample's main peak pI value is within ± 0.10 of mean pI value of main peak of reference standard. Sample electrophoretic profile is comparable to the electrophoretic profile of reference standard. | | | | | | | |

### CLAUSES

The following clauses define particular implementations of the present disclosure:
1. A formulation for nasal delivery comprising: 0.25 or 0.5 mg/mL foralumab, 3.4 mg/mL sodium acetate, 0.20 mg/mLpolysorbate 80 and 7.31 mg/mL sodium chloride.
2. The formulation of clause 1, wherein the formulation upon storage for three months at about 2 °C to about 8 °C has the following characteristics:
   a. being substantially free of microbial contamination;
   b. being substantially free of protein aggregates;
   c. retains at least 65% lymphocyte proliferation activity compared to a control.
3. The formulation of clause 2, wherein the formulation further comprises the following characteristics:
   d. retains at least 70% of T-cell activation activity compared to a reference;
   e. has a protein concentration of at least 90% of the starting concentration;
   f. has a pH of 5.5 ± 0.2.
4. The formulation of clause 1, wherein the formulation is a liquid.
5. The formulation of clause 1, wherein the volume of the formulation is 100 µL.
6.. A unit dose device for the intranasal administration of foralumab comprising a nasal atomization device comprising 0.25 or 0.5 mg/mL foralumab, 3.4 mg/mL sodium acetate, 0.20 mg/mL polysorbate 80, 7.31 mg/ mL sodium chloride.
7. The unit dose device of clause 6, wherein the device delivers a 100 µL dose of the formulation.
8. The unit dose device of clause 6, wherein the device delivers a dosage unit with a relative standard deviation (RSD) of no greater than 10%; no greater than 9%; no greater that 8%; no greater than 7%; than 6%; no greater than 5%; no greater that 4%; no greater than 3%.
9. The unit dose device of clause 6, wherein the nasal atomization device is an Aptar UniDose device.
10. The unit dose device of clause 9 wherein the delivered droplet size delivered is between 10 µm and 100 µm.
11. The unit dose device of clause 6, wherein the nasal atomization devise is a Gerresheimer device
12. The unit dose device of clause 10, wherein the delivered droplet size delivered is between 25 µm and 250 µm.
13. The unit dose devise of clause 6, wherein the delivered droplet size is between 0.1 µm and 1000 µm.
14. The formulation of clause 1, wherein the foralumab concentration in the formulation is within 3%, within 5%, or within 10 % of the starting concentration after store at about 2 °C to about 8 °C for at least about one month, at least about three months, at least about 6 months, at least about 12 months, at least about 18 months, at least about 2 years, at least about 3 years or at least about 5 years.
15. The formulation of clause 1, wherein the formulation contains less than 1%, less than 1.5%, less than 2% or less than 2.5% aggregation after store at about 2 °C to about 8 °C for at least about one month, at least about three months, at least about 6 months, at least about 12 months, at least about 18 months, at least about 2 years, at least about 3 years or at least about 5 years.
16. The formulation of clause 1, wherein the pH of the formulation remains within 0.1, 0.2 or 0.3 of starting value after store at about 2 °C to about 8 °C for at least about one month, at least about three months, at least about 6 months, at least about 12 months, at least about 18 months, at least about 2 years, at least about 3 years or at least about 5 years.
17. The formulation of clause 1, wherein the potency of the foralumab in the nasal anti-CD3 antibody formulation remains within 10%, within 15%, within 20%, within 25%, within 30%, or within 35% of the starting potency after storage at about 2 °C to about 8 °C (e.g., about 5 °C) for at least about one month, at least about three months, at least about 6 months, at least about 12 months, at least about 18 months, at least about 2 years, at least about 3 years or at least about 5 years.
18. A method of treating an autoimmune disease, an inflammatory disorder, a neurodegenerative disease or cancer in a subject in need thereof, comprising administering to the subject the formulation of clause 1.
19. The method of clause 18, wherein the disease or disorder is multiple sclerosis or Alzheimer's Disease

## Claims

1. A unit dose device for the intranasal administration of a liquid formulation comprising foralumab; wherein 10 µg-100 µg of foralumab is delivered per dose, in a volume of 100 µL of the formulation, and wherein the delivered droplet size is (a) between 10 µm and 100 µm or (b) between 25 µm and 250 µm.

2. The unit dose device of claim 1, wherein the device delivers a dose of 25 µg to 50 µg of foralumab per administration.

3. The unit dose device of claim 1 or 2, wherein the formulation has a pH of about 4 to 8.

4. The unit dose device of claim 1, 2 or 3, wherein the formulation is administered to one nostril or to both nostrils.

5. The unit dose device of any preceding claim, wherein the formulation is substantially free of protein aggregates.

6. A liquid formulation comprising an anti-CD3 antibody for use in treating or alleviating a symptom of an autoimmune disease, an inflammatory disorder, a neurodegenerative disease or cancer in a subject in need thereof, wherein the formulation is for nasal administration, and wherein 10 µg to 100 µg of the anti-CD3 antibody is administered per dose, in a volume of 100 µL of the formulation, and wherein the delivered droplet size is (a) between 10 µm and 100 µm or (b) between 25 µm and 250 µm, and wherein the anti-CD3 antibody comprises a heavy chain complementarity determining region (CDRH) 1 comprising the amino acid sequence of SEQ ID NO: 1, a CDRH2 comprising the amino acid sequence of SEQ ID NO: 3, a CDRH3 comprising the amino acid sequence of SEQ ID NO: 4, a light chain complementarity determining region (CDRL) 1 comprising the amino acid sequence of SEQ ID NO: 5, a CDRL2 comprising the amino acid sequence of SEQ ID NO: 6, and a CDRL3 comprising the amino acid sequence of SEQ ID NO: 7.

7. The formulation for use of claim 6, wherein the anti-CD3 antibody comprises a variable heavy chain amino acid sequence comprising the sequence of SEQ ID NO: 8 and a variable light chain amino acid sequence comprising the sequence of SEQ ID NO: 9.

8. The formulation for use of claim 6 or 7, wherein 25 µg to 50 µg of the anti-CD3 antibody is administered per dose.

9. The formulation for use of claim 6, 7 or 8, wherein the formulation has a pH of about 4 to 8.

10. The formulation for use of any of claims 6 to 9, wherein the formulation is administered to one nostril or to both nostrils.

11. The formulation for use of any of claims 6 to 10, wherein the anti-CD3 antibody is foralumab.

12. The formulation for use of claim 11, wherein the formulation is substantially free of protein aggregates.

13. A liquid formulation comprising an anti-CD3 antibody for use in treating a neurodegenerative disease or alleviating a symptom of a neurodegenerative disease in a subject in need thereof, wherein the formulation is for nasal administration; wherein 10 µg to 100 µg of the anti-CD3 antibody is delivered per administration; and wherein the delivered droplet size of the formulation is between 10 and 250 µm.

14. The formulation for use of claim 13, wherein the anti-CD3 antibody comprises a heavy chain complementarity determining region (CDRH) 1 comprising the amino acid sequence of SEQ ID NO: 1, a CDRH2 comprising the amino acid sequence of SEQ ID NO: 3, a CDRH3 comprising the amino acid sequence of SEQ ID NO: 4, a light chain complementarity determining region (CDRL) 1 comprising the amino acid sequence of SEQ ID NO: 5, a CDRL2 comprising the amino acid sequence of SEQ ID NO: 6, and a CDRL3 comprising the amino acid sequence of SEQ ID NO: 7.

15. The formulation for use of claim 13 or 14, wherein the anti-CD3 antibody comprises a variable heavy chain amino acid sequence comprising the sequence of SEQ ID NO: 8 and a variable light chain amino acid sequence comprising the sequence of SEQ ID NO: 9.

16. The formulation for use of claim 13, 14 or 15, wherein 25 µg to 50 µg of the anti-CD3 antibody is delivered per administration.

17. The formulation for use of any of claims 13 to 16, wherein the neurodegenerative disease is multiple sclerosis or Alzheimer's Disease.

18. The formulation for use of any of claims 13 to 17, wherein the anti-CD3 antibody is foralumab.

19. The formulation for use of any of claims 13 to 18, wherein the formulation is administered to one nostril or to both nostrils.

20. The formulation for use of any of claims 13 to 19, wherein the formulation has a pH of about 4 to 8.

21. The formulation for use of any of claims 13 to 20, wherein the formulation comprises 0.25 or 0.5 mg/mL of foralumab, 3.4 mg/mL sodium acetate, 0.20 mg/mL polysorbate 80 and 7.31 mg/mL sodium chloride.
